(19) Europäisches Patentamt
European Patent Office
Office européen des brevets

(11) Veröffentlichungsnummer: **0 206 060**
**B1**

(12) # EUROPÄISCHE PATENTSCHRIFT

(45) Veröffentlichungstag der Patentschrift: **18.05.88**

(21) Anmeldenummer: **86107754.3**

(22) Anmeldetag: **06.06.86**

(51) Int. Cl.⁴: **C 07 C 119/048**, C 07 C 118/02, C 08 G 18/77

(54) **Neue Diisocyanate, ein Verfahren zu ihrer Herstellung und ihre Verwendung als Aufbaukomponente bei der Herstellung von Polyurethankunststoffen oder von Polyurethan-Vorprodukten.**

(30) Priorität: **13.06.85 DE 3521126**

(43) Veröffentlichungstag der Anmeldung: **30.12.86 Patentblatt 86/52**

(45) Bekanntmachung des Hinweises auf die Patenterteilung: **18.05.88 Patentblatt 88/20**

(84) Benannte Vertragsstaaten: **BE DE FR GB IT**

(56) Entgegenhaltungen:
**EP-A-0 058 368**
**US-A-2 986 576**

**CHEMICAL ABSTRACTS, Band 77, Nr. 6, 7. August 1972, Seite 3, Zusammenfassung Nr. 34991d, Columbus, Ohio, US; B.F. MALICHENKO et al.: "Polyfluoroalkoxyphenylene diisocyanates and their derivatives"**
**CHEMICAL ABSTRACTS, NINTH COLLECTIVE INDEX, Bände 76-85, 1972-1976, Seite 3742C8, Spalte 3, American Chemical Society, Columbus, Ohio, US; "2,4-diisocyanato-1-(pentyloxy)- 37491-20-6, 77: 34991d"**

(73) Patentinhaber: **BAYER AG, Konzernverwaltung RP Patentabteilung, D-5090 Leverkusen 1 Bayerwerk (DE)**

(72) Erfinder: **Scholl, Hans Joachim, Dr., Am Feldrain 5, D-5000 Köln 80 (DE)**
Erfinder: **Pedain, Josef, Dr., Haferkamp 6, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

EP 0 206 060 B1

**Beschreibung**

Die Erfindung betrifft neue 1-Alkoxy-2,4-diisocyanatobenzole, ein Verfahren zu ihrer Herstellung durch Umsetzung von 1-Chlor-2,4-dinitrobenzol mit primären Alkoholen in Gegenwart von speziellen Katalysatorsystemen zu den entsprechenden 1-Alkoxy-2,4-dinitrobenzolen, Hydrierung dieser Dinitroverbindungen zu den entsprechenden Diaminen und deren anschließende Phosgenierung und die Verwendung der neuen Diisocyanate als Aufbaukomponente bei der Herstsllung von Polyurethankunststoffen nach dem Isocyanat-Polyadditionsverfahren oder bei der Herstellung von Polyurethan-Vorprodukten.

Diisocyanatobenzole, die höhere, d.h. 8 bis 18 Kohlenstoffatome aufweisende Alkylsubstituenten tragen, stellen besonders interessante Ausgangsmaterialien für die Herstellung von Polyurethankunststoffen dar, da es sich bei diesen Diisocyanaten um Flüssigkeiten eines niedrigen Dampfdrucks handelt, die beispielsweise im Vergleich zu den homologen Diisocyanatotoluolen in physiologischer Hinsicht weit weniger problematisch sind, wobei als weiterer Vorteil hinzukommt, daß die Alkylsubstituenten nicht nur den Diisocyanaten sondern auch den aus ihnen hergestellten Zwischenprodukten (z. B. den aus den Diisocyanaten hergestellten NCO-Prepolymeren) eine verbesserte Löslichkeit in wenig polaren Lösungsmitteln verleihen. Derartige, höhere Alkylreste als substituentenaufweisende Diisocyanatobenzole bzw. ein Verfahren zu ihrer Herstellung werden beispielsweise in der DE-AS-1 123 662 (= GB-PS-852 988 = US-PS-2 986 576) beschrieben. Das dem Verfahren dieser Vorveröffentlichung zugrundeliegende alkylsubstituierte Diaminobenzol wird gemäß US-PS-2 934 571 durch Dinitrierung des entsprechenden Alkylbenzols und Hydrierung des resultierenden, alkylsubstituierten Dinitrobenzols erhalten. Nachteilhaft bei dieser Verfahrensweise ist, daß die Dinitrierung der Alkylbenzole gemäß US-PS-2 934 571 selbst unter Verwendung eines Gemischs aus rauchender Salpetersäure und rauchender Schwefelsäure als Nitriersäure nur unvollständig gelingt, so daß streng difunktionelle Diisocyanate kaum erhältlich sind. Auf diesen Sachverhalt wird in derEP-B-0 058 368näher eingegangen. Die Autoren dieser Vorveröffentlichung machten sich zur Aufgabe die genannten Nachteile der Verfahrensweise gemäß DE-AS-1 123 662 bzw. US-PS-2 934 571 zu überwinden. Dies gelang durch Verwendung spezieller, als Homologengemische vorliegender Alkylbenzole als Ausgangsmaterial für die Nitrierung, wobei die als Ausgangsmaterialien dienenden Alkylbenzole Homologengemische darstellen, welche bei 1013 mbar einen Siedebereich von 10 bis 50°C, vorzugsweise 20 bis 30°C innerhalb des Temperaturbereichs von 270 bis 330°C aufweisen und wobei die Alkylsubstituenten gesättigte, geradkettige aliphatische Kohlenwasserstoffreste mit 8 bis 15, vorzugsweise 10 bis 13 Kohlenstoffatomen darstellen. Derartige Alkylbenzol-Homologengemische müssen in einem separaten Arbeitsgang durch Alkylierung von Benzol mit den entsprechenden, linearen Olefingemischen hergestellt werden, die ihrerseits durch eine Oligomerisation von Ethylen erhalten werden. Es kann daher gesagt werden, daß es sich bei den Diisocyanaten der EP-B-0 058 368 zwar um technisch interessante Ausgangsmaterialien für den Polyurethanchemiker handelt, daß jedoch die Herstellung der Diisocyanate technisch aufwendig ist.

Es war daher die der Erfindung zugrundeliegende Aufgabe, neue, substituierte Diisocyanatobenzole zur Verfügung zu stellen, die bezüglich ihrer Eigenschaften den Diisocyanaten gemäß EP-B-0 058 368weitgehend entsprechen, die jedoch auf technisch einfache Weise aus wohlfeilen Ausgangsmaterialien zugänglich sind.

Diese Aufgabe konnte durch die Bereitstellung der nachstehend näher beschriebenen erfindungsgemäßen Diisocyanate und das nachstehend näher beschriebene Verfahren zu ihrer Herstellung gelöst werden. Im übrigen wurde überraschenderweise festgestellt, daß die neuen, erfindungsgemäßen Diisocyanate im Vergleich zu den entsprechenden Diisocyanaten gemäß EP-B-0 058 368die vorteilhafte Eigenschaft aufweisen, daß aus ihnen hergestellte Vorprodukte für Polyurethane, beispielsweise ihre Mischtrimerisate mit 2,4-Diisocyanatotoluol eine vergleichsweise niedrigere Viskosität aufweisen. Gegenstand der Erfindung sind, gegebenenfalls Isomeren und/oder Homologengemische darstellende Diisocyanate der Formel

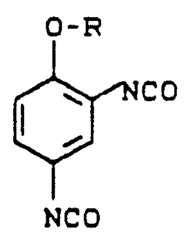

in welcher

R für einen Alkylrest mit 8 bis 18 Kohlenstoffatomen steht.

Gegenstand der Erfindung ist auch ein Verfahren zur Herstellung dieser Diisocyanate, welches dadurch gekennzeichnet ist, daß man

a) 1-Chlor-2,4-dinitrobenzol mit primären, gegebenenfalls Isomeren- und/oder Homologengemische darstellenden Alkoholen der Formel

R-OH

in Gegenwart mindestens eines Alkalihydroxids und mindestens eines quaternären Ammonium- oder Phosphoniumsalzes in gegebenenfalls Isomeren- und/oder Homologengemische darstellende 1-Alkoxy-2,4-dinitrobenzole der allgemeinen Formel

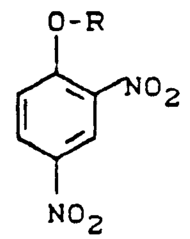

überführt,

b) die Dinitroverbindungen der Stufe a) in an sich bekannter Weise zu den entsprechenden Diaminen hydriert und

c) die Diamine der Stufe b) durch Phosgenierung in die Diisocyanate überführt.

Gegenstand der Erfindung ist schließlich auch die Verwendung der neuen, gegebenenfalls Isomeren- und/oder Homologengemische darstellenden Diisocyanate als Ausgangsmaterialien bei der Herstellung von Polyurethankunststoffen nach dem Isocyanat-Polyadditionsverfahren oder bei der Herstellung von Polyurethan-Vorprodukten.

Ausgangsmaterialien für das erfindungsgemäße Verfahren sind 1-Chlor-2,4-dinitrobenzol und primäre Alkohole der Formel R-OH.

Das 1-Chlor-2,4-dinitrobenzol kann in an sich bekannter Weise durch Dinitrierung von Chlorbenzol erhalten werden und fällt hierbei in einer Reinheit von mindestens 98 Gew.-% an. Dieses technische Produkt kann neben der Hauptkomponente (1-Chlor-2,4-dinitrobenzol) geringe Mengen an anderen, isomeren Chlordinitrobenzolen aufweisen, die jedoch die Durchführbarkeit des erfindungsgemäßen Verfahrens nicht beeinträchtigen, so daß im Rahmen der Erfindung unter "1-Chlor-2,4-dinitrobenzol" sowohl die chemisch reine Substanz als auch technisch reines 1-Chlor-2,4-dinitrobenzol mit einem Reinheitsgrad von mindestens 98 Gew.-% zu verstehen ist.

Bei den Alkoholen der Formel R-OH handelt es sich um primäre Alkohole, für welche R für einen Alkylrest mit 8 bis 18, vorzugsweise 8 bis 15 Kohlenstoffatomen steht, wobei diese Alkylreste sowohl linear als auch verzweigt sein können. Als Alkoholkomponente können beim erfindungsgemäßen Verfahren sowohl chemisch reine Substanzen als auch Isomeren- und/oder Homologengemische eingesetzt werden, deren Einzelkomponenten der genannten Formel entsprechen. Vorzugsweise werden die entsprechenden linearen primären Alkohole bzw. Alkoholgemische verwendet. Beispiele geeigneter Alkohole sind n-Octanol, n-Dodecanol, n-Pentadecanol, n-Octadecanol, die entsprechenden primären Alkanole mit verzweigten Alkylresten, beliebige Gemische derartiger Alkanole oder auch technische Gemische derartiger primärer Fettalkohole, wie sie beispielsweise als Waschmittelvorprodukte im Handel erhältlich sind. Hierzu gehören beispielsweise die von der Shell Chemie unter dem Warenzeichen Dobanol(R) angebotenen Alkoholgemische wie z. B. Dobanol 91, welches ein Gemisch linearer primärer Alkohole mit Kettenlängen von $C_9$ bis $C_{11}$ darstellt, Dobanol 23, welches ein Gemisch linearer primärer Alkohole mit Kettenlängen von $C_{12}$ bis $C_{13}$ darstellt, Dobanol 25, welches ein Gemisch linearer primärer Alkohole mit Kettenlängen von $C_{12}$ bis $C_{15}$ darstellt oder Dobanol 45, welches ein Gemisch primärer linearer Alkohole mit Kettenlängen von $C_{14}$ bis $C_{15}$ darstellt.

Die Stufe a) des erfindungsgemäßen Verfahren wird in Gegenwart mindestens eines Alkalihydroxids, d.h. in Gegenwart von Natrium- und/oder Kaliumhydroxid durchgeführt. Die Alkalimetallhydroxide werden vorzugsweise in einer Menge von 1 bis 3 Mol pro Mol 1-Chlor-2,4-dinitrobenzol eingesetzt, wobei die Hydroxide sowohl in Substanz als auch in Form wäßriger, vorzugsweise 40-50 gew.-%iger Lösung zum Einsatz gelangen. Die Verwendung von unterstöchiometrischen Mengen an Alkalihydroxid würde selbstverständlich zu Ausbeuteverlusten führen, während die Verwendung von mehr als 3-fach stöchiometrischen Mengen unwirtschaftlich und in chemischer Hinsicht sinnlos wäre.

Die Stufe a) des erfindungsgemäßen Verfahrens wird in Gegenwart von Katalysatoren durchgeführt. Geeignete Katalysatoren sind quaternäre Ammonium oder Phosphoniumsalze der Formel

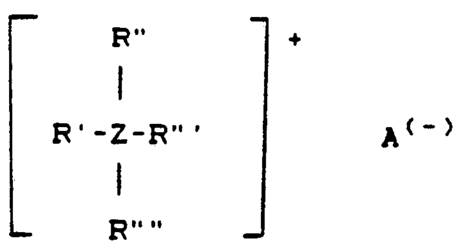

in welcher

Z für Stickstoff oder Phosphor steht,

R', R", R''' und R'''' für gleiche oder verschiedene Reste stehen und Alkylgruppen mit 1 bis 18 Kohlenstoffatomen bedeuten, wobei einer der Reste auch fur einen araliphatischen Rest mit 7 bis 15 Kohlenstoffatomen stehen kann, und wobei die Summe der Kohlenstoffatome der vier Reste vorzugsweise bei

12 bis 29 liegt, und A(-) für das Anion einer starken Mineralsäure, insbesondere für ein Chlorid- oder Bromidion steht.

Typische Beispiele geeigneter Katalysatoren sind N-Benzyl-N,N,N-triethyl-ammoniumchlorid oder -bromid, N-Benzyl-N-dodecyl-N,N-dimethyl-ammoniumchlorid oder -bromid, N,N,N,N-Tetra-n-hexyl-ammoniumchlorid oder -bromid, N-Benzyl-N,N,N-tri-n-octyl-ammoniumchlorid oder -bromid oder die diesen Ammoniumsalzen entsprechenden Phosphoniumsalze.

Die beispielhaft genannten quaternären Ammonium- oder Phosphoniumsalze werden bei der Durchführung der Stufe a) des erfindungsgemäßen Verfahrens vorzugsweise in Substanz oder in Form ihrer wäßrigen Lösungen (beispielsweise mit einem Feststoffgehalt von 30 bis 60 Gew.-%) und vorzugsweise in einer Menge von 1 bis 10 Mol-%, bezogen auf 1-Chlor-2,4- dinitrobenzol eingesetzt.

Die Stufe a) des erfindungsgemäßen Verfahrens wird vorzugsweise in Gegenwart eines inerten Lösungsmittels durchgeführt. Geeignete Lösungsmittel sind beispielsweise Tetrahydrofuran, Dioxan, Acetonitril, Benzol, Toluol, Xylol, Chlorbenzol, Dichlorbenzol, Chlortoluol, Methylenchlorid oder Gemische derartiger Lösungsmittel.

Zur Durchführung der Stufe a) des erfindungsgemäßen Verfahrens kann man beispielsweise so vorgehen, daß man das 1-Chlor-2,4-dinitrobenzol und den primären Alkohol in Gegenwart des als Festsubstanz oder als wäßrige Lösung eingesetzten Alkalimetallhydroxids und in Gegenwart eines quaternären Ammoniumsalzes der beispielhaft genannten Art in einem organischen Lösungsmittel der beispielhaft genannten Art als Reaktionsmedium rührt. Die Umsetzung erfolgt vorzugsweise unter Normaldruck innerhalb des Temperaturbereichs von 15 bis 50°C, vorzugsweise 20 bis 40°C. Es ist beispielsweise möglich, das erfindungsgemäße Verfahren in der Stufe a) so durchzuführen, daß man ein Gemisch aller Reaktanten und Hilfsmittel unter Rühren vorlegt und in das gerührte Gemisch eine Lösung des 1-Chlor-2,4-dinitrobenzols in einem Lösungsmittel der beispielhaft genannten Art allmählich einträgt. Der Fortgang der Umsetzung kann titrimetrisch verfolgt werden (Verbrauch des eingesetzten Alkalihydroxids durch die freigesetzte Salzsäure). Nach Beendigung der Stufe a) des erfindungsgemäßen Verfahrens wird die organische Phase neutral gewaschen und vorzugsweise ohne weitere Behandlung der Stufe b) des erfindungsgemäßen Verfahrens zugeführt. Grundsätzlich ist es jedoch auch möglich, das in der Stufe a) eingesetzte Lösungsmittel destillativ zu entfernen und das anfallende 1-Alkoxy-2,4-dinitrobenzol in einem anderen, beispielsweise in Ethanol oder Isopropanol gelöst der Stufe b) des erfindungsgemäßen Verfahrens zuzuführen.

Die hier beschriebene Herstellung von 1-Alkoxy-2,4-dinitrobenzolen eignet sich selbstverständlich auch für die Herstellung von 1-Alkoxy-2,4-dinitrobenzolen unter Verwendung von kurzkettigen Alkoholen. Die erfindungswesentliche Katalyse ist im übrigen auch bei anderen Austauschreaktionen anwendbar, bei denen chlorsubstituierte Aromaten, die aktivierend wirkende Substituenten wie z. B. Nitrogruppen aufweisen in die entsprechenden Alkoxy-substituierten Derivate überführt werden sollen.

Die Stufe b) des erfindungsgemäßen Verfahrens besteht in der an sich bekannten Hydrierung der Nitrogruppen zu den entsprechenden primären Aminogruppen beispielsweise unter Verwendung von Raney-Nickel als Hydrierungskatalysator bei beispielsweise 20-60°C und einem Wasserstoffdruck von beispielsweise 20 bis 40 bar. Nach Beendigung der Hydrierung können die Diamine, die der Formel

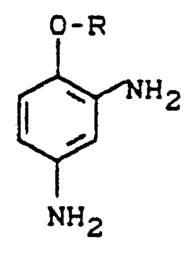

entsprechen durch übliche Aufarbeitungsverfahren aus der organischen Phase isoliert werden.

In der Stufe c) des erfindungsgemäßen Verfahrens werden die so erhaltenen Diamine einer an sich bekannten Phosgenierungsreaktion unterzogen. Hierzu wird beispielsweise das Diamin in einem Hilfslösungsmittel wie Chlorbenzol gelöst und in eine Lösung von Phosgen in Chlorbenzol unter Rühren und Kühlen bei -20 bis +5°C, vorzugsweise -10 bis 0°C eingetropft (Kaltphosgenierung), wonach das Reaktionsgemisch unter fortlaufendem Rühren und Einleiten von Phosgen auf eine Endtemperatur von 80 bis 130°C, vorzugsweise 90 bis 110°C erhitzt wird, um das zunächst gebildete Carbamidsäurechlorid in das angestrebte Diisocyanat zu überführen (Heißphosgenierung). Anschließend wird das Reaktionsgemisch in an sich bekannter Weise aufgearbeitet. Selbstverständlich kann die Überführung der Diamine in die erfindungsgemäßen Diisocyanate auch nach beliebigen anderen, an sich bekannten Phosgeniermethoden erfolgen. Die erfindungsgemäßen Diisocyanate stellen flüssige Substanzen dar, die keine Kristallisationsneigung erkennen lassen, die, bedingt durch ihren geringen Dampfdruck, bei Raumtemperatur keinen nennenswerten Eigengeruch aufweisen, und die herstellungsbedingt keine störenden Restanteile an Monoisocyanaten aufweisen. Es handelt sich, in Abhängigkeit von der Natur des eingesetzten Alkohols R-OH (und in Abhängigkeit von der Reinheit des eingesetzten 1-Chlor-2,4-dinitrobenzols) entweder um chemisch weitgehend einheitliche Substanzen oder um Isomeren- und/oder Homologengemische von Diisocyanaten der eingangs genannten allgemeinen Formel. Die erfindungsgemäßen Diisocyanate stellen wertvolle

Ausgangsmaterialien zur Herstellung von Polyurethankunststoffen nach dem Isocyanat-Polyadditionsverfahren dar. Für diesen Verwendungszweck können die erfindungsgemäßen Diisocyanate anstelle der bislang eingesetzten aromatischen Diisocyanate eingesetzt werden. Dies bedeutet, daß sich die erfindungsgemäßen Diisocyanate hervorragend zur Herstellung von beispielsweise Polyurethanschaumstoffen, -elastomeren, -klebstoffen, -dispersionen, -beschichtungen oder -lacken nach den an sich bekannten Verfahren unter Mitverwendung der an sich bekannten Reaktionspartner und Hilfsstoffe eignen. Die erfindungsgemäßen Diisocyanate eignen sich auch ausgezeichnet zur Herstellung von Polyurethan-Vorprodukten. Hierunter sind beispielsweise die aus der Polyurethanchemie an sich bekannten NCO-Prepolymeren oder die aus der Polyurethanchemie an sich bekannten modifizierten Polyisocyanate, insbesondere die durch teilweise Trimerisierung der Isocyanatgruppen erhältlichen, Isocyanuratgruppen aufweisenden Polyisocyanate zu verstehen. So eignen sich die erfindungsgemäßen Diisocyanate beispielsweise sehr gut zur Herstellung von Isocyanato-isocyanuraten, wobei die erfindungsgemäßen Diisocyanate als alleinige Ausgangsdiisocyanate oder im Gemisch mit anderen Diisocyanaten, beispielsweise mit 2,4-Diisocyanatotoluol einer an sich bekannten Trimerisierungsreaktion zugeführt werden. Die Mischtrimerisate der erfindungsgemäßen Diisocyanate mit 2,4-Diisocyanatotoluol weisen beispielsweise eine niedrigere Viskosität auf als entsprechende Mischtrimerisate von alkylsubstituierten Diisocyanaten gemäß EP-B-0 058 368 mit, 2,4-Diisocyanatotoluol. Die erfindungsgemäßen Diisocyante stellen außerdem wertvolle Zwischenprodukte zur Herstellung von Schädlingsbekämpfungsmitteln dar.

Die nachfolgenden Beispiele dienen der näheren Erläuterung der Erfindung. Alle Prozentangaben beziehen sich, falls nicht anders lautend erwähnt, auf Gewichtsprozente.

Für die dünnschichtchromatographischen Untersuchungen wurden DC-Fertigplatten, Kieselgel 60 F-254 der Firma Merck AG verwendet.

## Beispiele

### Beispiel 1

In diesem Beispiel wird ein Homologengemisch synthetischer, primärer Fettalkohole eingesetzt, deren Alkylketten eine Länge von 9 bis 11 C-Atomen bei einer mittleren Kettenlänge von etwa 10 C-Atomen aufweisen (Dobanol(R) 91 der Firma Shell Chemie). Das Homologengemisch siedet gemäß ASTM D 1078 bei 1013 mbar zwischen ca. 225 und 248° C.

a) Alkoxylierung von 1-Chlor-2,4-dinitrobenzol

176 g Fettalkoholgemisch werden mit 200 g Toluol, 16 g einer 50 %-igen wäßrigen Lösung von N-Benzyl-N-dodecyl-N,N-dimethyl-ammoniumchlorid und 220 g einer 50 %-igen Kaliumhydroxidlösung unter Rühren vermischt. Nachfolgend läßt man bei 20-30° C eine Lösung von 203 g 1-Chlor-2,4-dinitrobenzol in 500 g Toluol innerhalb von 2 h zutropfen und rührt 3 h bei 40° C nach. Die Reaktionsmischung wird nachfolgend mit Wasser neutral gewaschen und die so erhaltene Lösung von 1-Alkoxy-2,4-dinitrobenzol in Toluol direkt der Hydrierung zugeführt. Nach dünnschichtchromatographischen Untersuchungen war 1-Chlor-2,4-dinitrobenzol vollkommen umgesetzt (Laufmittel: 50 Gew.-Teile Petrolether, 50 Gew.-Teile Ether).

Vergleichsversuch zur Stufe a)

Gemäß Beispiel 1 wurde die Reaktion ohne Zusatz an Ammoniumsalz durchgeführt. Vergleichende dünnschichtchromatographische Untersuchungen zeigten einen nur geringen Umsatz an. Als Hauptbestandteil des Reaktionsgemischs war unverändertes 1-Chlor-2,4-dinitrobenzol nachzuweisen.

b) Hydrierung des 1-Alkoxy-2,4-dinitrobenzolgemisches in Toluol

1015 g Toluol-Lösung aus Stufe a) werden in einem Rührautoklaven mit 40 g Raney-Nickel versetzt. Bei 20-60° C und 20-40 bar Wasserstoffdruck wird bis zum Ende der Wasserstoffaufnahme gerührt. Anschließend wird entspannt, vom Katalysator abfiltriert und das Niedrigsiedergemisch Toluof/Wasser abdestilliert. Das entstandene Rohamingemisch wird anschließend unter vermindertem Druck destilliert. Auf diese Weise können 213 g 1-Alkoxy-2,4-diaminobenzol-Gemisch als bei 170-180° C/1 mbar siedende Fraktion erhalten werden (Gesamtausbeute: 80 %).

| Analyse (%) | C | H | N |
|---|---|---|---|
| gefunden: | 72,9 | 10,7 | 10,5 |
| Theorie: | 72,7 | 10,6 | 10,6 |

(bezogen auf $C_{16}H_{28}N_2O$)

c) Phosgenierung von 1-Alkoxy-2,4-diaminobenzolgemisch

In einem 4 l Vierhalskolben, mit Rührer, Thermometer, Gaseinleitungsrohr und Rückflußkühler versehen, werden 800 g trockenes Chlorbenzol vorgelegt. Unter Rühren und Kühlung (-10° C) werden ca. 320 g Phosgen einkondensiert, anschließend läßt man unter Kühlung bei -10 bis -5° C 213 g 1-Alkoxy-2,4-diaminobenzolgemisch aus Stufe b) in 300 g Chlorbenzol gelöst, zutropfen. Unter weiterem Einleiten von Phosgen wird die Lösung anschließend auf 40° C erwärmt und nachfolgend nach Maßgabe einsetzender Schaumentwicklung langsam bis 70° C gesteigert. Danach erwärmt man zügig auf Rückfluß und phosgeniert bis zum Ende der Chlorwasserstoffentwicklung nach (ca. 30 min). Das überschüssige Phosgen wird mit Stickstoff ausgeblasen und die Lösung im Vakuum eingedampft. Das entstandene Rohisocyanatgemisch wird anschließend unter vermindertem Druck destilliert. Auf diese Weise können 244 g 1-Alkoxy-2,4-diisocyanatobenzol-Gemisch als bei 169 bis 178° C/1 mbar siedende Fraktion erhalten werden (Ausbeute: 96 %).

| Analyse (%) | NCO | C | H | N |
|---|---|---|---|---|
| gefunden: | 26,2 | 68,5 | 7,7 | 8,9 |
| Theorie: | 26,4 | 68,3 | 7,6 | 8,9 |

(bezogen auf $C_{18}H_{24}N_2O_3$)

**Beispiel 2** (Verwendungsbeispiel)

750 Gew.-Teile des Diisocyanats gemäß Beispiel 1 werden mit 7,5 Gew.-Teilen eines Katalysators der Formel

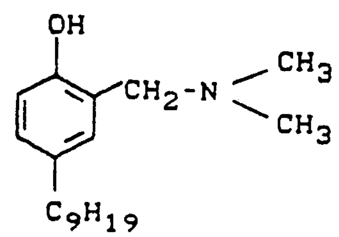

(Kondensationsprodukt von p-Isononylphenol, Formaldehyd und Dimethylamin) während 1 h bei 50° C gerührt. Die hierbei ablaufende Trimerisierungsreaktion wird bei einem NCO-Gehalt des Reaktionsansatzes von 16 % durch Zugabe von 4 Gew.-Teilen Toluolsulfonsäure-methylester abgebrochen. Man erhält so ein Isocyanuratgruppen aufweisendes Polyisocyanat als nahezu farblose, klare Flüssigkeit mit einem NCO-Gehalt von 16 % und einer Viskosität bei 50° C von ca. 20 mPa.s. Der Gehalt an freiem, monomerem Diisocyanat liegt bei 1,8 %.

Das Produkt ist klar in aromatischen oder aliphatischen Lösungsmitteln löslich und kann beispielsweise als Lösung in Lackbenzin zusammen mit den üblichen Hydroxylgruppen aufweisenden Ausgangsmaterialien für Zweikomponenten-Polyurethanlacke wie z. B. Hydroxylgruppen aufweisenden Alkydharzen, Folyesterharzen oder Polyacrylatharzen zu Lackschichten hoher Beständigkeit verarbeitet werden.

**Beispiel 3** (Verwendungsbeispiel)

Ein Gemisch aus 180 Gew.-Teilen 2,4-Diisocyanatotoluol und 20 Gew.-Teilen des Diisocyanats gemäß Beispiel 1 wird in 200 Gew.-Teilen eines Gemischs aus Xylol und Butylacetat (Gewichtsverhältnis = 1:1) gelöst und mit 0,4 Gew.-Teilen eines Trimerisierungskatalysators versetzt. Als Trimerisierungskatalysator dient eine Mannichbase, die im wesentlichen aus einem Gemisch von Verbindungen der Formeln

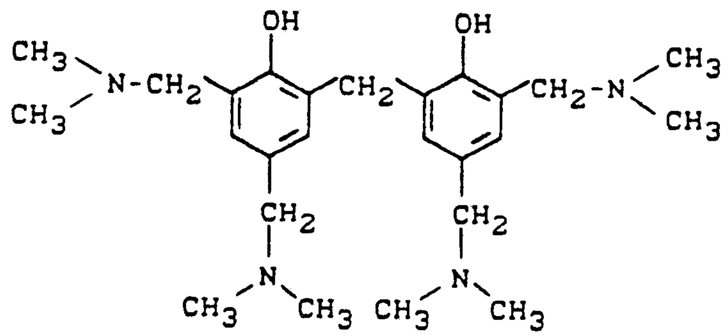

und

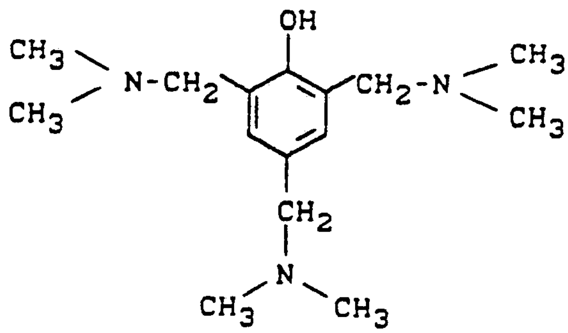

besteht.

Man rührt 10 Stunden bei 50°C und gibt nochmals 0,4 Gew.-Teile des Katalysatorgemischs zu und rührt erneut während 10 Stunden bei 50°C. Anschließend wird die Trimerisierungsreaktion durch Zugabe von 0,9 Gew.-Teilen Toluolsulfonsäuremethylester abgebrochen.

Auf diese Weise erhält man eine stabile, klare, farblose Lösung eines Isocyanuratgruppen aufweisenden Polyisocyanats mit einem NCO-Gehalt von 8,5 %, einem Gehalt an freiem Diisocyanatotoluol von 0,09 % und einem Gehalt an Diisocyanat gemäß Beispiel 1 von 0,3 % (jeweils bezogen auf Lösung). Die Viskosität der Lösung bei 23°C liegt bei 1100 mPa.s.

Aufgrund der niedrigen Viskosität der Polyisocyanatlösung eignet sich diese hervorragend als Härterkomponente in lösungsmittelarmen Zweikomponenten-Polyurethanlacken. Das Isocyanuratgruppen aufweisende Polyisocyanat zeichnet sich im übrigen durch eine gute Verträglichkeit mit den üblichen Bindemittelkomponenten wie z. B. Nitrozellulose oder Zelluloseacetobutyrat aus.

**Patentansprüche**

1. Gegebenenfalls Isomeren- und/oder Homologengemische darstellende Diisocyanate der Formel

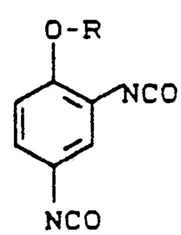

in welcher

R für einen Alkylrest mit 8 bis 18 Kohlenstoffatomen steht.

2. Verfahren zur Herstellung von, gegebenenfalls Isomeren- und/oder Homologengemische darstellenden Diisocyanaten gemäß Anspruch 1, dadurch gekennzeichnet, daß man

a) 1-Chlor-2,4-dinitrobenzol mit primären, gegebenenfalls Isomeren- und/oder Homologengemische darstellenden Alkoholen der Formel

R-OH

in Gegenwart mindestens eines Alkalihydroxids und mindestens eines quaternären Ammonium- oder Phosphoniumsalzes in gegebenenfalls Isomeren- und/oder Homologengemische darstellende 1-Alkoxy-2,4-dinitrobenzole der allgemeinen Formel

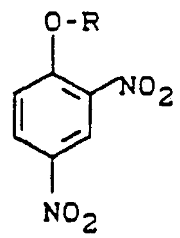

überführt,

b) die Dinitroverbindungen der Stufe a) in an sich bekannter Weise zu den entsprechenden Diaminen hydriert und

c) die Diamine der Stufe b) durch Phosgenierung in die Diisocyanate überführt.

3. Verwendung der, gegebenenfalls Isomeren- und/oder Homologengemische darstellenden Diisocyanate gemäß Anspruch 1 als Aufbaukomponente bei der Herstellung von Polyurethankunststoffen nach dem Isocyanat-Polyadditionsverfahren oder bei der Herstellung von Polyurethan-Vorprodukten.

**Claims**

1. Diisocyanates corresponding to the following formula optionally in the form of isomeric and/or homologous mixtures:

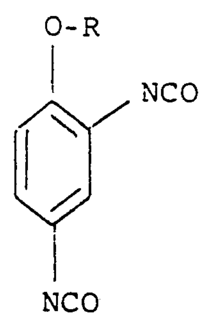

wherein

R stands for an alkyl group with 8 to 12 carbon atoms.

2. Process for the preparation of diisocyanates optionally consisting of isomeric and/or homologous mixtures according to claim 1, characterised in that

a) 1-chloro-2,4-dinitrobenzene is converted into 1-alkoxy-2,4-nitrobenzenes corresponding to the following general formula optionally consisting of isomeric and/or homologous mixtures:

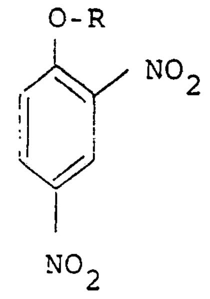

by means of primary alcohols corresponding to the formula

R-OH

optionally consisting of isomeric and/or homologous mixtures in the presence of at least one alkali metalhydroxide and at least one quaternary ammonium or phosphonium salt,

b) the dinitro compounds of stage a) are hydrogenated to the corresponding diamines in known manner and

c) the diamines of stage b) are converted into the diisocyanates by phosgenation.

3. Use of the diisocyanates according to claim 1 optionally consisting of isomeric and/or homologous mixtures as starting components for the preparation of polyurethane plastics by the isocyanate polyaddition process or for the preparation of polyurethane starting materials.

**Revendications**

1. Diisocyanates constituant éventuellement des mélanges d'isomères et/ou d'homologues, de formule:

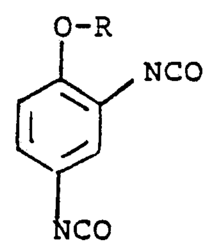

dans laquelle

R représente un radical alcoyle ayant 6 à 18 atomes de carbone.

2. Procédé de préparation de diisocyanates selon la revendication 1, constituant éventuellement des mélanges d'isomères et/ou d'homologues, caractérisé en ce que:

a) on convertit du 1-chloro-2,4-dinitrobenzène avec des alcools de formule

R-OH

qui constituent éventuellement des mélanges d'isomères et/ou d'homologues, en présence d'au moins un hydroxyde alcalin et en présence d'au moins un sel d'ammonium ou de phosphonium quaternaire, en des 1-alcoxy-2,4-dinitrobenzènes de formule générale:

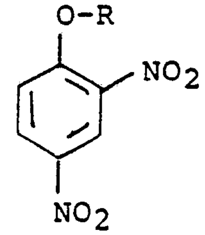

qui constituent éventuellement des mélanges d'isomères et/ou d'homologues,

b) on hydrogène les dinitrocomposés du stade a) d'une manière connue en elle-même en les diamines correspondantes et

c) on convertit les diamines du stade b) par phosgénation en les diisocyanates.

3. Utilisation des diisocyanates selon la revendication 1, qui constituent éventuellement des mélanges d'isomères et/ou d'homologues, comme composants d'édification dans la fabrication de matières synthétiques en polyuréthanes par le procédé de polyaddition à l'isocyanate, ou dans la fabrication de produits précurseurs de polyuréthanes.